**Europäisches Patentamt**

**European Patent Office**

**Office européen des brevets**

(19)

(11) Publication number : **0 445 854 B1**

## (12) EUROPEAN PATENT SPECIFICATION

(45) Date of publication of patent specification :
**18.08.93 Bulletin 93/33**

(51) Int. Cl.[5] : **A61K 7/16**

(21) Application number : **91200283.9**

(22) Date of filing : **11.02.91**

(54) **Oral compositions.**

(30) Priority : **21.02.90 GB 9003915**

(43) Date of publication of application :
**11.09.91 Bulletin 91/37**

(45) Publication of the grant of the patent :
**18.08.93 Bulletin 93/33**

(84) Designated Contracting States :
**AT BE CH DE DK ES FR GB GR IT LI NL SE**

(56) References cited :
**US-A- 4 100 269**
**US-A- 4 152 418**
**J. CHEM. RES. SYNOP., vol. 3, 1982, page 73,**
**Saldford, GB; A. DYER et al.: "A new synthesis**
**of sodium zincosilicate"**

(73) Proprietor : **UNILEVER N.V.**
**Weena 455**
**NL-3013 AL Rotterdam (NL)**
(84) **BE CH DE DK ES FR GR IT LI NL SE AT**
Proprietor : **UNILEVER PLC**
**Unilever House Blackfriars P.O. Box 68**
**London EC4P 4BQ (GB)**
(84) **GB**

(72) Inventor : **Coulson, Bryony Emma**
**UNILEVER Research Port Sunlight Lab.,**
**Bebington**
**Wirral, Merseyside L63 3JW (GB)**
Inventor : **Creeth, Andrew Martin**
**UNILEVER Research Port Sunlight Lab.,**
**Bebington**
**Wirral, Merseyside L63 3JW (GB)**

(74) Representative : **van Gent, Jan Paulus et al**
**Unilever N.V., Patent Division Postbus 137**
**NL-3130 AC Vlaardingen (NL)**

## Description

The present invention relates to oral compositions for the care of the teeth. More particularly, it relates to oral compositions for the care of the teeth, which compositions comprise a particular abrasive dental cleaning agent, which also delivers a therapeutically active agent to the teeth.

It is common practice nowadays to include an abrasive dental cleaning agent in oral compositions for the care of the teeth. Its primary function is to assist, by its abrasive action, the mechanical action of the toothbrush when brushing the teeth to remove food debris, plaque and tartar from the teeth. Usually, the abrasive dental cleaning agent is selected from abrasive particulate materials such as silicas, aluminas, calcium carbonates, dicalciumphosphates, insoluble sodium metaphosphates etc.

It is also well-known to include therapeutic agents in an oral composition for the care of the teeth. Thus, it is known from USP 4,100,269 to include a zinc compound such as zinc citrate as an antimicrobial agent in oral compositions to reduce or prevent the formation of plaque on the teeth by the action of the zinc ions on the oral microorganisms.

We have now surprisingly found, that particular alkalimetal zincosilicates are not only effective abrasive dental cleaning agents but also can deliver zinc ions to prevent or reduce the formation of plaque as well as calculus on the teeth.

In other words, they are effective both as an abrasive agent as well as an antiplaque and anti-calculus agent. These alkalimetal zincosilicates are known per-se, e.g. from European patent application 0 027 736. They are represented by the following general formula

$M_n Zn_p Si_r O_s$, in which M = alkalimetal, n is 1-2, p is 1-2.1, r is 1-3 and s is 4-8.

Typical examples are:

$Na_2 Zn Si O_4$

$Na_2 Zn Si_3 O_8$

$Na_{1.69} Zn Si O_4$

$Na_2 Zn_{1.27} Si O_4$

$Na Zn_{1.5} Si_2 O_6$

$Na Zn_2 Si_{2.5} O_{7.5}$

$Na Zn_{2.1} Si_{2.33} O_7$

$Na Zn_{1.86} Si_{2.37} O_{7.1}$

$Na Zn_{1.4} Si_{1.7} O_{5.2}$

$Na Zn_{1.96} Si_{2.4} O_{7.3}$

$Na Zn_{1.6} Si_{1.8} O_{5.3}$

Of these sodium zincosilicates, $Na_2 Zn Si O_4$ is the preferred compound. This compound dissolves in aqueous solution between pH6 and pH4, and dissolution would thus be localised in the oral environment in the more acidic regions near bacterial colonies, thus releasing zinc at the site of its biostatic action.

Its structure, as well as that of $Na_2 Zn Si_3 O_8$ has been described in Sov. Phys.-Crystallogr. 9 (1965), 795 and ibidem 24 (1979) 674; these materials were found to have a tectosilicate structure, composed of tetrahedrally coordinated zinc and silicon atoms. The sodium cations were located in small pores inside the framework.

Their hardness was in the region of 4-6 on the Mohs' scale.

The alkalimetal zincosilicates of the present invention are included in the oral compositions in an amount of generally from 0.1-50% by weight, usually from 0.1-30% by weight and preferably from 0.1-20% by weight. They can be prepared by the methods as described in the aforementioned references.

The oral composition of this invention will, of course, also contain other ingredients commonly used to formulate such products, depending on the form of the oral product. For instance, in the case of an oral product in the form of a dentifrice cream or paste the product will comprise an humectant-containing liquid phase and a binder or thickener which acts to maintain the particulate solid abrasive in stable suspension in the liquid phase. A surfactant and a flavouring agent are also usual ingredients of commercially acceptable dentifrices.

Humectants commonly used are glycerol and sorbitol syrup (usually comprising an approximately 70% solution). However, other humectants are known to those in the art including propylene glycol, lactitol, and hydrogenated corn syrup. The amount of humectant will generally range from about 10 to 85% by weight of the dentifrice. The liquid phase can be aqueous or nonaqueous.

Likewise numerous binding or thickening agents have been indicated for use in dentifrices, preferred ones being sodium carboxymethylcellulose and xanthan gum. Others include natural gum binders such as gum tragacanth, gum karaya and gum arabic, Irish moss, alginates and carrageenans. Silica thickening agents include the silica aerogels and various precipitated silicas. Mixtures of binders may be used. The amount of binder included in a dentifrice is generally between 0.1 and 10% by weight.

EP 0 445 854 B1

It is usual to include a surfactant in a dentifrice and again the literature discloses a wide variety of suitable materials. Surfactants which have found wide use in practice are sodium lauryl sulphate, sodium dodecylbenzene sulphonate and sodium lauroylsarcosinate. Other anionic surfactants may be used as well as other types such cationic, amphoteric and non-ionic surfactants. Surfactants are usually present in an amount of from 0.5 to 5% by weight of the dentifrice.

Flavours that are usually used in dentifrices are those based on oils of spearmint and peppermint. Examples of other flavouring materials used are menthol, clove, wintergreen, eucalyptus and aniseed. An amount of from 0.1% to 5% by weight is a suitable amount of flavour to incorporate in a dentifrice.

The oral compositions of the invention may also comprise a proportion of a supplementary abrasive agent such as silica, alumina, hydrated alumina, calcium carbonate, anhydrous dicalcium phosphate, dicalcium phosphate dihydrate and water-insoluble sodium metaphosphate.

The oral composition of the invention may include a wide variety of optional ingredients. These include an anti-plaque agent such as an antimicrobial compound for example chlorhexidine or 2,4,4'-trichloro-2'-hydroxy-diphenyl ether, or a zinc salt (see EP-A-161 898); an anti-tartar ingredient such as a condensed phosphate e.g. an alkali metal pyrophosphate, hexametaphosphate or polyphoshate, (see US-A-4 515 772 and US-A-4 627 977) or zinc citrate (see US-A-4 100 269); sweetening agent such as saccharin; an opacifying agent, such as titanium dioxide; a preservative, such as formalin, or a colouring agent.

Fluorine-containing anti-caries compounds such as sodium fluoride and sodium monofluorophosphate can also be included. Other examples of known fluorine-containing anti-caries agents are stannous fluoride, amine fluorides and other fluoride-ion sources e.g. as described in U.S. patent 4,684,518 (Parran et al.).

For a fuller discussion of the formulation of oral composition reference is made to Harry's Cosmeticology, Seventh Edition, 1982, Edited by J.B. Wilkinson and R.J. Moore, pages 609 to 617.

The invention also relates to a method of combating dental plaque which consists in applying to the teeth, such as by brushing, an oral composition according to the invention.

The invention is further illustrated by the following Examples. Percentage and parts are by weight.

Examples

Three samples of sodium zincosilicate $Na_2ZnSiO_4$ were prepared by the method, given in European Patent application 0 027 736, using the following conditions:

Three solutions were prepared, having the following compositions:

Solution A:     3.93 g sodium metasilicate dissolved in 25 g water.
Solution B:     89.0 g sodium hydroxide dissolved in 90 g water, then 15 g ZnO dissolved with stirring.
Solution C:     24.85 g sodium silicate solution.

Solution B was warmed to temperature T/°C, then solution A followed by solution C were added slowly. The mixture was stirred at temperature T for 5 hours, then the resulting solid was filtered and dried at 95°C.

| | T/°C | Yield |
|---|---|---|
| Sample 1 | 98-105° | 28.2 g |
| Sample 2 | 110° | 32.0 g |
| Sample 3 | 90° | 27.5 g |

Particle sizes and Dentine Abrasion values

Particle sizes were determined using a Malvern Light scattering instrument.

Table 1 shows the results of the size measurements together with Dentine Abrasion values (DAV) carried out on 10% aqueous slurries of the samples. All these samples met the British Standard requirement for toothpastes that abrasivity as measured by DAV must not exceed 200. These zincosilicates displayed adequate and flexible abrasivities.

Dissolution tests

For these tests, 0.5 g of sample 3 was suspended in 50 g water and stirred using a magnetic stirring bar. The solution was pH-statted at set pH values by addition of 0.975 M $HNO_3$.

The pH was varied to mimic the changes when toothpaste is diluted into saliva. Therefore the suspension was statted at pH 7 for 2 hours. The pH was then set to pH 6 and the amount of acid added recorded as a function of time. The pH was then returned to pH 7 before repeating the above procedure at pH 5.

Samples were removed 300 s after the pH was set, diluted with water and centrifuged. An aliquot of the supernatant was taken and diluted into 0.1 M $HNO_3$ and the level of zinc measured by atomic absorption. The amount of dissolved zinc released into the original zincosilicate suspension during the 300 s at the set pH is recorded in Table 2.

## Table 1

| Sample no. | DAV | Average particle size Malvern |
|---|---|---|
| 1 | 56.6 | 36.0 |
| 2 | 54.0 | 36.7 |
| 3 | 60.1 | 30.8 |

## Table 2

Amount of zinc released during 300 s at set pH

| pH | $[Zn^{2+}]/10^{-3}M$ |
|---|---|
| 6 | 0.86 |
| 5.5 | 3.74 |

## Claims

1. An oral composition comprising a zinc ions releasing compound, characterised in that the zinc ions releasing compound is an alkalimetal zincosilicate having the general formula $M_n\,Zn_p\,Si_r\,O_s$, in which M is an alkalimetal, n = 1-2, p = 1-2.1, r = 1-3 and s = 4-8.

2. An oral composition according to claim 1, characterized in that M = Na, n= 2, p = 1, r = 1 and s = 4.

3. An oral composition according to claim 1 or 2, characterised in that the alkalimetal zincosilicate is present in an amount of 0.1 - 50% by weight of the composition.

## Patentansprüche

1. Eine orale Zusammensetzung, die eine Zinkionen freisetzende Verbindung umfaßt, dadurch gekennnzeichnet, daß die Zinkionen freisetzende Verbindung ein Alkalimetallzinksilikat mit der allgemeinen Formel $M_n\,Zn_p\,Si_r\,O_s$ ist, worin M ein Alkalimetall, n = 1-2, p = 1-2.1, r = 1-3 und s = 4-8 ist.

2. Eine orale Zusammensetzung nach Anspruch 1, dadurch gekennnzeichnet, daß M = Na, n = 2, p = 1, r = 1 und s = 4 ist.

3. Eine orale Zusammensetzung nach Anspruch 1 oder 2, dadurch gekennnzeichnet, daß das Alkalimetallzinksilikat in einer Menge von 0.1 - 50 Gew% der Zusammensetzung vorliegt.

**Revendications**

1. Une composition orale comprenant un composé libérant des ions de zinc, caractérisée en ce que le composé libérant des ions de zinc est un silicate de zinc et de métal alcalin ayant la formule générale $M_n Zn_p Si_r O_s$, dans laquelle M est un métal alcalin, n est égal à 1 - 2, p est égal à 1 - 2,1, r est égal à 1 - 3 et s est égal à 4 - 8.

2. Une composition orale selon la revendication 1, caractérisée en ce que M est Na, n est égal à 2, p est égal à 1, r est égal à 1 et s est égal à 4.

3. Une composition selon la revendication 1 ou 2, caractérisée en ce que le silicate de zinc et de métal alcalin est présent dans une quantité de 0,1 - 50 % en masse de la composition.